Europäisches Patentamt

European Patent Office

Office européen des brevets

(11) Publication number: **0 243 178 B1**

(12) **EUROPEAN PATENT SPECIFICATION**

(45) Date of publication of patent specification :
19.06.91 Bulletin 91/25

(51) Int. Cl.[5]: **A61L 27/00, A61L 25/00,**
**// A61K35/28**

(21) Application number: 87303548.9

(22) Date of filing : 22.04.87

(54) A marrow/collagen/mineral matrix for bone defect repair.

(30) Priority : 22.04.86 US 855004

(43) Date of publication of application :
28.10.87 Bulletin 87/44

(45) Publication of the grant of the patent :
19.06.91 Bulletin 91/25

(84) Designated Contracting States :
AT BE CH DE ES FR GB GR IT LI LU NL SE

(56) References cited :
EP-A- 0 171 176
EP-A- 0 197 693
JP-A-58 058 041
US-A- 3 949 073
US-A- 4 440 750

(56) References cited :
CHEMICAL ABSTRACTS, vol. 87, no. 17, 24th
October 1977, page 76, abstract no. 127420p,
Columbus, Ohio, US; A. STABHOLZ et al.:
"Effect of fluoride on autogenous iliac can-
cellous bone and marrow transplants in surgi-
cally created intrabony pockets in dogs", & J.
PERIODONTOL. 1977, 48(7), 413-17

(73) Proprietor : COLLAGEN CORPORATION
2500 Faber Place
Palo Alto, California 94303 (US)

(72) Inventor : Piez, Karl A.
1120 Bay Laurel Drive
Menlo Park California 94025 (US)
Inventor : Parr, Jack E.
Rural Route 2, Box 133 B
North Webster Indiana 46551 (US)

(74) Representative : Goldin, Douglas Michael et al
J.A. KEMP & CO. 14, South Square Gray's Inn
London WC1R 5EU (GB)

EP 0 243 178 B1

## Description

### Technical Field

The invention relates to bone repair materials, in particular to bone repair materials which are composites of living and nonliving components. Specifically, the invention concerns the use of autogeneic bone marrow in combination with collagen and mineral extenders as a bone repair matrix.

### Background Art

The use of autogeneic bone particles and/or bone marrow is widespread and well known in the art. See, for example, Salama, R., et al, J Bone Joint Surg (Br) (1973) 55 : 402-417. Bone marrow has also been combined with biodegradable ceramic for periodontal defect repair (Levin, M.P., et al, J Biomed Mater Res (1975) 9 : 183-195). The marrow or cancellous bone appears quite effective in mediating repair. However, the use of marrow alone is limited because of its fluid nature.

While it seems clear that bone or bone marrow derived from the same individual, or, at worst, from an individual closely related, is helpful in repairing bone injuries or defects, it is a problem to obtain sufficient material to effect the desired repair. Due to the immunogenicity of foreign tissue, it is necessary to restrict the bone marrow or bone chips to those which are derived from the same individual or from genetically close relatives. With such a limited source, the scarcity-of-supply problem becomes acute. It would therefore be helpful to find a method to extend the healing effects of these biological materials by providing a suitable carrier. One carrier which shows promise is collagen per se, which has been used in bone repair in view of its role as the major organic substituent of bone.

The use of collagen preparations for the repair of bone defects has also been extensively reported. The use of Collagenfleece®, in particular, which is a freeze-dried, pepsin-treated preparation from pig skin, sterilized by gamma irradiation (as described in US 4,066,083) has been reported by Krekeler, B.G., et al, J Oral Surg (1981) 10 : suppl. 1 : 151 ; Joos, U., et al, Biomaterials (1980) 1 : 23-26 ; Zetzmann, D., et al, Schweiz Mschr Sabnhelik (1982) 92 : 119 ; and Springorum, H.W., et al, Z Orthop (1977) 115 : 686. Other collagen preparations were used by Jaffee, A., et al, Arch Oral Biol (1978) 23 : 415 ; ihid (1982) 27 : 999, and by Cucin, R.L., et al, Ny State J Med (1979) 1856. The use of atelopeptide fibrillar bovine collagen as a composition for conductive bone repair was disclosed in U.S. serial No. 752,447, filed 5 July 1985, assigned to the same assignee, and incorporated herein by reference. In addition, collagen has been used in conjunction with a factor extractable from bone which mediates inductive bone repair as disclosed by Jeffries in U.S. 4,394,370 and in U.S. Serial No. 664,158, filed 24 October 1984, assigned to the same assignee and incorporated herein by reference. Bone marrow has also been admixed with collagen alone (U.S. Serial No. 829,809, filed 14 February 1986, assigned to the same assignee and incorporated herein by reference).

While bone marrow or cancellous bone may be extended with a collagen suspension for some purposes, the strength and handling properties of compositions based on collagen alone as a matrix or carrier are not always suitable. Attempts have been made to extend cancellous bone implants using calcium phosphate minerals ; one such attempt appears to have been successful (Moore, D.C., M.S. Thesis, U.C.-Davis (1985)). Therefore, the addition of a mineral component to alter the physical properties of the collagen-based matrix containing bone marrow may be desirable.

Reports of attempts to use a collagen suspension/mineral combination are numerous. For example, Lemons, J., et al., reported at the Second World Congress of Biomaterials in Washington, D.C., 27 April-1 May 1984, on attempts to utilize collagen along with commercial hydroxyapatite and calcium phosphate to repair artificially created lesions in rabbits. The use of these mixtures did not result in reunion of the lesions. A control experiment using fresh autogenous bone, however, was successful in producing a union. Similarly, Levy, P., et al, J Periodontol (1981), : 303-306, were unsuccessful in their attempts to utilize collagen/mineral gel implants to repair intrabony defects in root canals of canine or monkey teeth. Gross, B.C., et al., Oral Surg (1980), 49 : 21-26, reported limited success in using mixtures of reconstituted lyophilized calfskin collagen in admixture with a hydroxyapatite preparation to induce bone growth through sub-periosteal implants in monkeys. Various others have reported use of forms of collagen which clearly contain telopeptides, a major source of immunogenicity of collagen, in combination with minerals in bone repair. See, for example, Hayashi, K. et al., Arch Orthop Traumat Surg (1982) 99 : 265-269 ; Battista, U.S. Patent 4,349,490 (using a hydrated gelatin) ; Cruz, Jr., U.S. Patent 3,767,437 (using a calcium-precipitated form of collagen) ; and Battista, et al., U.S. Patent 3,443,261 (utilizing, in addition to calcium phosphate, a "new form" of collagen which contains microcrystals of aggregated tropocollagen units.

Miyata, et al., U.S. Patent 4,314,380, utilized a mineral backbone prepared directly by treatment of animal bone to remove all organic materials, which was then coated with an atelopeptide collagen. Japanese Application J58/058041, published 6 April 1983, discloses a spongy porous calcium phosphate material having pores treated with atelopeptide collagen. The collagen derives from collagen-in-solution having a concentration of not more than 2% by weight. The Japanese application reports the advance of osteoblasts into the pores of the material and new bone growth. European Patent Application, Publication No. 030583, published 24 June 1981, discloses use of Collagenfleece® in admixture with hydroxyapatite in bone repair. This collagen material is a commercial product, is obtained from animal hide by proteolytic digestion of noncollagenous impurities, and is lyophilized and sterilized by gamma irradiation. This collagen preparation forms a soft membrane-like material but does contain telopeptides and is partially degraded by the processing.

The invention provides compositions wherein autogeneic bone marrow is extended with a collagen preparation, and its handling properties are improved by the addition of a mineral component.

## Disclosure of the Invention

The invention provides a composition for repair of bone defects that permits the advantages inherent in natural tissue bone marrow to be utilized while overcoming the problems of shortage of supply and difficulty in handling. It has been found that compositions containing suitable reconstituted fibrillar collagen preparations as carriers are capable of performing the function of extending the supply of compatible tissue to effect bone repair efficiently and in a manner comparable to the effects of these tissues alone. By the addition of a calcium phosphate mineral component, the handling properties of the composition are made suitable for repair of stress-bearing defects as well as those which are not subject to compression or torsion.

Accordingly, in one aspect, the invention is directed to a method to mediate the repair of bone defects which utilizes a composition containing, in addition to autogeneic bone marrow, an extender and strengthener which consists of reconstituted fibrillar atelopeptide collagen and calcium phosphate mineral particles along with sufficient physiological saline to malleability. In another aspect, the invention relates to compositions which are useful in this method, and to processes for preparing these compositions.

## Modes of Carrying Out the Invention

## A. The Bone Marrow Component

The compositions for use in the method of the invention comprise matrices which consist essentially of an autogeneic bone marrow component and a collagen/mineral extender/strengthener component. The autogeneic bone marrow is preferably derived from the same individual who bears the defect to be repaired, or, if this is not possible, from an individual sufficiently closely related genetically that the materials derived from this individual are not immunogenic in the recipient. It is recognized that, except for the same individual or an identical twin, the degree of genetic relatedness represents a continuum, and that methods for predicting in advance whether or not sufficient close relationship is present to prevent immunogenicity are not entirely precise. However, current practice does operate within these parameters and attempts to evaluate genetic matching for surgical transplantation procedures, have been made with varying degrees of success. In general, as used herein, "autogeneic" refers to material either derived from the same person (or his twin) or from an individual judged by standard and commonly practiced techniques and criteria to be sufficiently closely related to provide a workable source of such material.

The methods of obtaining this evaluation of "autogeneic" character and/or excising bone narrow from such individuals are standard in the art and do not form part of the invention. The bone marrow, prepared in these routine ways, is mixed immediately before implantation with the collagen/mineral extender/strengthener mixture. The bone marrow may be used as removed.

## B. The Collagen

The collagen portion of the preparation is a nonimmunogenic form of a reconstituted fibrillar preparation, such as, for example, commercially available preparations used for soft tissue repair. These preparations include Zyderm® Collagen Implant (ZCI), which is available from Collagen Corporation, Palo Alto, CA, in concentrations of 35 mg/ml and 65 mg/ml. In general, these collagen preparations are prepared from animal skins, although any source of predominantly type I collagen can be used. The preparation will include treatment with a suitable proteolytic enzyme to remove the telopeptide portions extending beyond the triple helical segments, which telopeptide portions are responsible for the native cross-linking between helices and for at least a portion of the immunogenicity of the preparation.

In a suitable procedure, a mammalian skin preparation, preferably bovine skin, is cleaned, depilated, and ground or minced to form a finely divided preparation which is solubilized under nondenaturing conditions by dispersing it in an aqueous medium and digesting with a proteolytic enzyme other than a collagenase, preferably an enzyme that is active at low pH. Dilute acid solutions of, for example, HCl or of carboxylic acids, such as acetic, malonic, or lactic acids, are used at low temperature with a pH normally in the range of 1.5-5, depending on the enzyme used. A preferred procedure is to disperse the comminuted tissue in HCl to a concentration of 1-5 g/l at a pH of about 2 at 20°C. After the tissue is dispersed, the enzyme is added and the mixture is incubated to permit the enzyme to digest the telopeptide and other solubiliz- able components of the tissue. Suitable enzymes for the digestion of the telopeptides which do not attack the triple helical portion of the collagen include pepsin, papain and trypsin, preferably pepsin, with an enzyme concentration in the range of 0.1%-10% by weight based on the collagen content of the tissue. The incubation period can last from about 2 days to 2 weeks and the process of solubilization may be monitored by determining the viscosity of the solution. Once the viscosity reaches a substantially constant level, the solubilization is complete, and the enzyme is deactivated and removed.

After denaturation of the enzyme, the solution is treated to remove the denatured enzyme and the digested portions of the tissue by various techniques, and combinations thereof including, for example, dialysis, sedimentation, or filtration. The soluble components including the collagen are segregated from sedimented or filtered solids and concentrated, optionally fractionated by precipitation or ion exchange chromatography, and further concentrated to produce a substantially pure atelopeptide collagen solution. Collagen is quite soluble in weak acid, such as 0.01 N HCl, and typical concentration levels of the collagen in the solution are 1-10 mg/ml.

The collagen in solution is reconstituted to a fibrillar form by neutralizing the solution at reduced temperatures, preferably about 10-25°C., preferably under hypotonic conditions relative to physiological ionic strength. The neutralizing solution may be added directly or, preferably, by dialysis of the solubilized collagen against it. Ionic strengths of about 0.03-0.1, preferably 0.06, are used, and the pH is raised by adding an appropriate base or buffer such as disodium phosphate or sodium hydroxide to a level at which the collagen in solution reaggregates into fibrils. Fibril formation occurs under these conditions at a pH in the range of about 5-10, and the final pH is preferably in the range of 7-8. The duration of the fibril formation step is normally in the range 1/2-18 hours.

Optionally, the reconstituted atelopeptide collagen gel suspension may be cross-linked with a cross-linking agent such as, for example, various aldehydes such as formaldehyde, acetaldehyde, glyoxalpyruvic aldebyde, and dialdebyde starch, preferably glutaraldehyde. During the cross-linking reaction, the collagen concentration is kept at about 1-10 mg/ml, preferably 2-5 mg/ml. Following cross-linking, the reaction may be quenched with compounds which have functional groups that react with the functional groups of the cross-linking agent to form water soluble adducts. In particular, compounds containing free amino groups, such as, for example, glycine, are usable in this respect. The excess cross-linking agent may also be removed by washing.

The collagen suspension that results (with or without cross-linking) is typically a suspension in aqueous solution at a concentration of 10-100 mg/ml,

preferably about 30-70 mg/ml. A favorable suspension medium is isotonic saline, but other buffer solutions or aqueous solutions which permit the collagen suspension to be stabilized are also acceptable.

The foregoing general preparation procedures are typical of those used to prepare suitable collagen for the composition of the invention. However, the specific procedure used is not significant so long as the resulting preparation is substantially free of the telopeptide portions, has been reconstituted, is fibrillar, and is basically pure collagen uncontaminated with materials coexisting with the collagen in its native environment.

Thus, "reconstituted" collagen refers to collagen which has been disassembled into individual native triple helical molecules brought into solution and then regrouped into "fibrillar" forms. In this form, the fibrils consist of long, thin collagen molecules staggered relative to one another by multiples of about one-fourth their length. This results in a banded structure similar to native fibrillar collagen which can be further aggregated into fibers.

One suitable collagen component is an atelopeptide collagen which is reconstituted into fibrillar form and supplied as a gel. Such reconstituted atelopeptide collagen gels are available commercially as Zyderm® Collagen Implant (ZCI) in preparations containing 35 mg/ml collagen or 65 mg/ml collagen in saline, manufactured by Collagen Corporation, Palo Alto, California. For use in the compositions of the inventions the ZCI preparations are used without lidocaine or other sedative drugs. As used herein, "ZCI" refers to the aqueous collagen dispersion, rather than to the collagen component per se.

## C. The Mineral Component

As used herein, "calcium phosphate mineral" materials refers to those materials composed of $Ca^{+2}$ and phosphate ions, regardless of the microstructure, protonation status of the phosphate, or extent of hydration. Calcium phosphate mineral materials include a variety of forms, such as the commercially available forms of tricalcium phosphate (TCP), for example, Synthograft® tricalcium phosphate, or of hydroxyapatite (HA) such as Periograf®, Alveograf®, OrthoMatrix™ HA-1000™, or OrthoMatrix™ HA-500™ hydroxyapatite particulate preparations. The hydroxyapatite or tricalcium phosphate may also be prepared by known methods, such as those disclosed by Termine, et al., Arch Biochem Biophys (1970) 140 : 307-325, or by Hayashi, K. et al., Arch Orthop Trauma Surg (supra). The preparations may have varying degrees of porosity and various densities. Halogenated analogs of calcium phosphate, such as calcium fluorophosphate are also included. Any biocompatible calcium phosphate mineral may be used, but mixtures of TCP and HA are preferred.

It should be noted that the mineral preparations used in the invention may have a range of porosities, and the porosity of course affects the density of the material. Commercially available calcium phosphate mineral preparations vary in bulk density in the range of 0.5-3.16 g/ml.

## D. The Mixture

The matrix portion of the compositions of the invention are preferably formed by simple mixing of the suspensions of the bone marrow, with a mixture previously prepared from the suspension of the fibrillar collagen and the calcium phosphate mineral. (In the alternative, other permutations may be used ; for example, the autogeneic material may first be mixed with the collagen and the mineral then added ; however, as the autogeneic material is usually supplied fresh from a donor, it is advantageous to have the remainder of the composition mixed in advance.)

The suspensions of the autogeneic material and the collagen/mineral may be mixed in a volume ratio containing as little as 5% of the marrow. It is known in the art that the autogeneic material is effective when supplied alone if kept in the defect, and the object of the invention is to reduce the amount of this material required and to improve its handling properties. It is thus apparent that an upper limit on the percentage of the autogeneic component is entirely arbitrary. For definiteness, however, it is considered that a range of of 5-50% by volume of marrow in admixture with the collagen/mineral suspension provides a sufficient decrease in the need for the autogeneic material to represent a contribution to the ability of the art to effect bone defect repair. Since the collagen suspension used to prepare the composition may vary severalfold in collagen concentration, and since the mineral component of the collagen/mineral matrix may vary over a sixfold range in density, this results in a considerable range of solid compositions based on dry weight. The final composition must be of such consistency so as to form a malleable mass, and the foregoing volume percentages assume that both the marrow and the mixture of collagen/mineral are supplied as suspensions of such concentrations that this is the result. The effective range of solid ingredients is therefore fairly wide.

The collagen/mineral mixture itself is prepared to be a malleable composition with sufficient collagen suspension to fully wet the mineral. When the collagen is supplied as a suspension of reasonable concentration, for example 3-7%, such as 3.5% or 6.5%, and the-mineral is supplied in dry form, weight ratios of the collagen suspension to the mineral of 80 : 30 to 30 : 70 will be workable depending on the porosity of the mineral component, both with respect to supplying the correct amount of fluid and the correct amount of collagen to the finished mixture. Of course, the collagen and mineral could also be mixed while dry, and fluid added to the mixture to give an equivalent composition. Since the collagen concentration is small, 3 to 7%, the weight ratio of mineral to fluid will also be about 80 : 20 to 30 : 70. The collagen by dry weight will then be in the range of 0.9 to 5.6 parts per 100.

The preferred ratios depend on a number of factors, including the nature of the bone materials used, the nature of the defect, the size of the defect, the metabolism of the subject, and judgment of the practitioner repairing the defect. As set forth above. the compositions of the invention comprise a wide range of ratios of bone materials to the collagen/mineral carrier.

In addition to the matrix materials, the compositions as used in the repair of the defects may also contain minor amounts of other ingredients, such as binders, buffers, medicaments, excipients and the like.

## E. Use of the Compositions

The compositions of the invention are implanted into a bone defect using standard surgical procedures typified by those used in supplying bone marrow or cancellous bone preparations alone. Such procedures are well known to orthopedic and dental surgery practitioners and are applied using the generally understood practices of the these professions.

Defects which are suitable subjects for implantation by the compositions of the invention include bone fractures, congenital bone defects, surgically created bone defects, bone structures requiring supplementation, such as alveolar ridge augmentation, and periodontal defects. Any defect which is a suitable substrate for filling with autogeneic cancellous bone, presently the material of choice, may be suitable for the composition of the invention, except that the increased availability of materials in the composition of the invention may make possible the treatment of defects which were too large and require too much material to permit filling with autogeneic bone.

In summary, the methods employ compositions which contain a matrix consisting essentially of a suspension of a bone material which comprises bone marrow, in admixture with a suspension of an atelopeptide pure reconstituted fibrillar collagen mixed with a calcium phosphate mineral. The bone marrow must be autogeneic as defined above ; that is, they must be derived from the same individual or from an individual sufficiently closely related that the preparations are nonimmunogenic in the recipient. The collagen and mineral portion, on the other hand, may be derived from any source. The removal of the telopeptides diminishes the immunogenicity of the collagen sufficiently that even xenogeneic sources may be used ; it is known that calcium phosphate minerals such as HA and TCP are biocompatible. The reconstituted fibrillar nature of the collagen preparation permits conduction of the bone growth which is initiated by the bone marrow in the composition ; the physical properties of the mineral permit more convenient handling and the use of the matrix in a wider range of defects than bone marrow alone. The mixture mimics cancellous bone but is more readily available.

The following examples are to illustrate the invention, but do not limit its scope.

## Example 1

### Preparation of Marrow/Collagen/Mineral Matrix

A mixture is prepared as follow : 4.8 cc of 65 mg/ml ZCl were thoroughly mixed with 2.5 g of the TCP/HA mixture (70% porosity, d = 0.6 g/ml, 4.2 cc). The volume of the resulting mixture was 5.6 cc. It contains about 4% collagen, 34% mineral and 62% saline by weight. To this mixture was added 1.4 cc of bone marrow, to obtain 7 cc of composition (20% of the composition by volume). The resulting composition is thus about 16% marrow, 3% collagen, 52% saline and 29% mineral by weight.

## Example 2

### Use of the Matrix in Bone Repair

The matrix prepared in Example 1 is compared to a matrix of reconstituted fibrillar collagen with mineral but without marrow, and a matrix of reconstituted fibrillar collagen with bone marrow in effectiveness as a bone graft material using a canine model.

The model is as follows : Eighteen adult mongrel dogs (2-5 years old, 20-30 kg) are verified to have mature skeletal structure by radiography and divided into three groups of 6 dogs each for the study. For surgery, each dog is anesthetized kith halothane and a 2.5 cm segment of the distal ulna, taken a standard distance from the styloid process is excised. The segments of the ulna are then connected with an intermedullary pin, and the test bone graft material implanted in the defect. The soft tissue and skin are closed in a standard manner.

In addition, a 9.5 × 20 mm defect is made in the cancellous bone of the proximal humerus with a water cooled trephine (Carb biopsy needle), this defect is also filled with the test bone graft material, and the soft tissue and skin are closed in a standard manner.

When bone marrow is to be a part of the test bone graft material, it is obtained by aspiration from the ipsilateral femur. Its cell count in the marrow of the various cell types is also obtained.

After recovery, the dogs are returned to their runs for routine housing and observation without external fixation of the operated limb or any restraint on activity.

At four week intervals, the dogs are anesthetized and radiographs taken to assess the amount of osteogenesis, degree of fusion, and extent of modeling. At various intervals, serum samples are taken and bone fluorescent labeling is effected by injection of appropriate dye, specifically tetracycline, calcein green, xylenol orange or alazarin red.

After 24 weeks, the dogs are sacrificed by injection of barbiturate and the repaired bone defects assessed by histology and by mechanical testing. Histology is performed by examination of thin slices embedded in methyl methacrylate : mechanical valuation is by Burstein-Frankel torque tester (Burstein, A.H., et al, J Biomechanics (1971) 4 : 155-158). Mechanical strength is based on load to failure, displacement to failure, load/displacement slope, and energy absorption.

The results show the preparation of Example 1 to be effective in mediating the regeneration of usable bone in both the ulna and humerus defects.

## Claims

**Claims for contracting States DE, GB, FR, IT, NL, SE, LI, CH, BE, AT and LU.**

1. A composition for the repair of defects in stress-bearing or nonstress-bearing bone, which comprises a matrix consisting essentially of autogeneic bone marrow in admixture with a collagen/mineral preparation containing reconstituted fibrillar atelopeptide collagen and a calcium phosphate mineral component and sufficient fluid to obtain a malleable preparation.

2. A composition according to claim 1 wherein the matrix is 5-50% autogeneic bone marrow volume.

3. A composition according to claim 1 or 2 wherein the collagen/mineral preparation is 20-70% mineral and 30-80% fibrillar collagen suspension.

4. A composition according to any one of the preceding claims wherein the collagen is supplied as a suspension of 30-70 mg/ml.

5. A composition according to any one of the preceding claims wherein the mineral is selected from hydroxyapatite (HA), tricalcium phosphate (TCP), or mixtures thereof having bulk densities of 0.5-3.16 g/cc.

6. A method of preparing a matrix consisting essentially of an autogeneic bone marrow, reconstituted fibrillar atelopeptide collagen and a calcium phosphate mineral component which comprises mixing autogeneic bone marrow with a previously prepared mixture of collagen and mineral.

7. A composition according to any one of claims 1 to 5 for use in a method for effecting the repair of defects in stress-bearing or nonstress-bearing bone, which comprises implanting the composition into a bone defect.

**Claims for the contracting States GR, ES.**

1. A method of preparing a matrix consisting essentially of an autogeneic bone marrow, reconstituted fibrillar atelopeptide collagen and a calcium phosphate mineral component which comprises mixing autogeneic bone marrow with a previously prepared mixture of collagen and mineral.

2. A method according to claim 1 wherein the matrix is 5-50% autogeneic bone marrow volume.

3. A method according to claim 1 or 2 wherein the collagen/mineral preparation is 20-70% mineral and 30-80% fibrillar collagen suspension.

4. A method according to any one of the preceding claims wherein the collagen is supplied as a suspension of 30-70 mg/ml.

5. A method according to any one of the preceding claims wherein the mineral is selected from hydroxyapatite (HA), tricalcium phosphate (TCP), or mixtures thereof having bulk densities of 0.5-3.16 g/cc.

6. A composition prepared according to the method of any one of claims 1 to 5 for use in a method for effecting the repair of defects in stress-bearing or nonstress-bearing bone, which comprises implanting the composition into a bone defect.

**Ansprüche**

**Patentansprüche für die Vertragstaaten DE, GB, FR, IT, NL, SE, LI, CH, BE, AT, LU.**

1. Stoffzusammensetzung zur Heilung oder Reparatur von Defekten in belasteten oder nichtbelasteten Knochen mit einer Matrix, die im wesentlichen besteht aus autogenetischem Knochenmark, dem ein Kollagen/Mineral-Präparat zugemischt ist, welches rekonstituiertes feinfaseriges Atelopeptid-Kollagen und eine Calcium-Phosphat-Mineralkomponente enthält sowie hinreichend Fluid, um eine knetbare Substanz zu erhalten.

2. Stoffzusammensetzung gemäß Anspruch 1, wobei die Matrix 5 bis 50% autogenetisches Knochenmark enthält, bezogen auf das Volumen.

3. Stoffzusammensetzung nach einem der Ansprüche 1 oder 2, wobei das Kollagen/Mineral-Material 20 bis 70% Mineral und 30 bis 80% feinfaseriges Kollagen als Suspension enthält.

4. Stoffzusammensetzung nach einem der vorhergehenden Ansprüche, wobei das Kollagen als Suspension von 30 bis 70 mg/ml hinzugefügt ist.

5. Stoffzusammensetzung nach einem der vorhergehenden Ansprüche, wobei als Material Hydroxyapatit (HA) oder Tricalciumphosphat (TCP) oder Mischungen davon vorgesehen sind mit Volumendichten von 0,5 bis 3,1 g/c$^3$.

6. Verfahren zum Anfertigen einer Matrix, im wesentlichen bestehend aus autogenetischem Knochenmark, rekonstituiertem feinfaserigen Atelopeptid-Kollagen und einer Calcium-Phosphat-Mineralkomponente, wobei autogenetisches Knochenmark mit einer zuvor angefertigten Mischung aus Kollagen und Mineral gemischt wird.

7. Stoffzusammensetzung nach einem der Ansprüche 1 bis 5 zur Verwendung bei einem Verfahren zum Heilen oder Reparieren von Defekten in belasteten oder nichtbelasteten Knochen, wobei die Stoffzusammensetzung in den Knochendefekt implantiert wird.

**Patentansprüche für die Vertragstaaten GR, ES.**

1. Verfahren zum Anfertigen einer Matrix, im wesentlichen bestehend aus autogenetischem Knochenmark, rekonstituiertem feinfaserigen Atelopeptid-Kollagen und einer Calcium-Phosphat-Mineralkomponente, wobei autogenetisches Knochenmark mit einer zuvor angefertigten Mischung aus Kollagen und Mineral gemischt wird.

2. Verfahren gemäß Anspruch 1, wobei die Matrix 5 bis 50% autogenetisches Knochenmark enthält, bezogen auf das Volumen.

3. Verfahren nach einem der Ansprüche 1 oder 2, wobei das Kollagen/Mineral-Material 20 bis 70% Mineral und 30 bis 80% feinfaseriges Kollagen als Suspension enthält.

4. Verfahren nach einem der vorhergehenden Ansprüche, wobei das Kollagen als Suspension von 30 bis 70 mg/ml hinzugefügt ist.

5. Verfahren nach einem der vorhergehenden Ansprüche, wobei als Material Hydroxyapatit (HA) oder Tricalciumphosphat (TCP) oder Mischungen davon vorgesehen sind mit Volumendichten von 0,5 bis 3,1 g/c$^3$.

6. Stoffzusammensetzung, die erhalten ist nach einem Verfahren gemäß einem der Ansprüche 1 bis 5 zur Verwendung bei einem Verfahren zum Heilen oder Reparieren von Defekten in belasteten oder nichtbelasteten Knochen, wobei die Stoffzusammensetzung in den Knochendefekt implantiert wird.

## Revendications

**Revendications pour les Etats contractants DE, FR, GB, IT, LI, NL, SE, LI, CH, BE, AT, LU.**

1. Composition pour la réparation de défauts dans un os soumis à un effort ou non soumis à un effort, caractérisée en ce qu'elle comprend une matrice consistant essentiellement en moelle d'os autogénique en mélange avec une préparation de collagène/composé minéral contenant du collagène atélopeptide fibrillaire reconstitué et un composant minéral de type phosphate de calcium et suffisamment de liquide pour obtenir une préparation malléable.

2. Composition suivant la revendication 1, caractérisée en ce que la matrice comprend 5 à 50% en volume de moelle d'os autogénique.

3. Composition suivant la revendication 1 ou la revendication 2, caractérisé en ce que la préparation de collagène/composé minéral comprend 20 à 70% de composé minéral et 30 à 80% de suspension de collagène fibrillaire.

4. Composition suivant l'une quelconque des revendications 1 à 3, caractérisée en ce que le collagène est fourni sous forme d'une suspension de 30 à 70 mg/ml.

5. Composition suivant l'une quelconque des revendications 1 à 4, caractérisée en ce que le composé minéral est choisi parmi l'hydroxyapatite (HA), le phosphate tricalcique (TCP) ou leurs mélanges ayant des densités en vrac de 0,5 à 3,16 g/cm$^3$.

6. Procédé pour préparer une matrice comprenant essentiellement une moelle d'os autogénique, un collagène atélopeptide fibrillaire reconstitué et un composant minéral de type phosphate de calcium, caractérisé en ce qu'il comprend le mélange de moelle d'os autogénique avec un mélange préalablement préparé de collagène et d'un composé minéral.

7. Composition suivant l'une quelconque des revendications 1 à 5 utile dans un procédé pour effectuer la réparation de défauts dans un os soumis à un effort ou non soumis à un effort, caractérisée en ce que cette composition est implantée dans un défaut osseux.

**Revendications pour les Etats contractants : GR, ES.**

1. Procédé pour préparer une matrice comprenant essentiellement une moelle d'os autogénique, un collagène atélopeptide fibrillaire reconstitué et un composant minéral de type phosphate de calcium, caractérisé en ce qu'il comprend le mélange de moelle d'os autogénique avec un mélange préalablement préparé de collagène et d'un composé minéral.

2. Procédé suivant la revendication 1, caractérisé en ce que la matrice comprend 5 à 50% en volume de moelle d'os autogénique.

3. Procédé suivant la revendication 1 ou la revendication 2, caractérisé en ce que la préparation de collagène/composé minéral comprend 20 à 70% de composé minéral et 30 à 80% de suspension de collagène fibrillaire.

4. Procédé suivant l'une quelconque des revendications 1 à 3, caractérisé en ce que le collagène est fourni sous forme d'une suspension 30 à 70 mg/ml.

5. Procédé suivant l'une quelconque des revendications 1 à 4, caractérisé en ce que le composé minéral est choisi parmi l'hydroxyapatite (HA), le phosphate tricalcique (TCP) ou leurs mélanges ayant des densités en vrac de 0,5 à 3,16 g/cm$^3$.

6. Composition préparée suivant le procédé de l'une quelconque des revendications 1 à 5 utile dans un procédé pour effectuer la réparation de défauts dans un os soumis à un effort ou non soumis à un effort, caractérisée en ce que cette composition est implantée dans un défaut osseux.